(19) Europäisches Patentamt
European Patent Office
Office européen des brevets

(11) Veröffentlichungsnummer: **0 158 012**
A1

(12) # EUROPÄISCHE PATENTANMELDUNG

(21) Anmeldenummer: 85100843.3

(22) Anmeldetag: 25.05.81

(51) Int. Cl.⁴: **C 07 D 513/04**
C 07 D 498/04, C 07 D 487/04
//(C07D513/04, 285:00, 235:00),
(C07D513/04, 277:00, 235:00),
(C07D498/04, 271:00, 235:00),
(C07D487/04, 249:00, 235:00),
(C07D498/04, 263:00, 235:00),
(C07D487/04, 235:00, 235:00)

(30) Priorität: 29.05.80 DE 3020421

(43) Veröffentlichungstag der Anmeldung:
16.10.85 Patentblatt 85/42

(84) Benannte Vertragsstaaten:
AT BE CH DE FR GB IT LI LU NL SE

(60) Veröffentlichungsnummer der früheren
Anmeldung nach Art. 76 EPÜ: 0 041 215

(71) Anmelder: BAYER AG
Konzernverwaltung RP Patentabteilung
D-5090 Leverkusen 1 Bayerwerk(DE)

(72) Erfinder: Meyer, Horst, Dr.
Henselweg 11
D-5600 Wuppertal 1(DE)

(72) Erfinder: Horstmann, Harald, Dr.
Claudiusweg 19
D-5600 Wuppertal 1(DE)

(72) Erfinder: Möller, Eike, Dr.
Pahlkestrasse 37
D-5600 Wuppertal 1(DE)

(72) Erfinder: Garthoff, Bernward, Dr.
Händelstrasse 22
D-4010 Hilden(DE)

(54) **Imidazoazolacrabonylverbindungen und ihre Verwendung als Zwischenprodukte für die Herstellung von Arzneimittel.**

(57) Imidazoazolcarbonylverbindungen der Formel

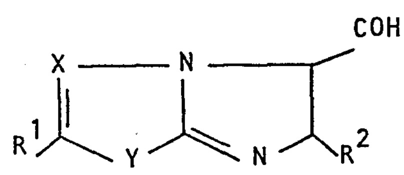

in welcher
X, Y, $R^1$ und $R^2$ dic in der Beschreibung angegebene Bedeutung haben, sind wertvolle Zwischenprodukte für die Herstellung von Arzneimitteln.

EP 0 158 012 A1

Croydon Printing Company Ltd

0158012

BAYER AKTIENGESELLSCHAFT 5090 Leverkusen, Bayerwerk
Konzernverwaltung RP
Patentabteilung Si/Th-c

# Imidazoazolcarbonylverbindungen und ihre Verwendung als Zwischenprodukte für die Herstellung von Arzneimittel

Die vorliegende Erfindung betrifft neue Imidazoazolcarbonylverbindungen der allgemeinen Formel

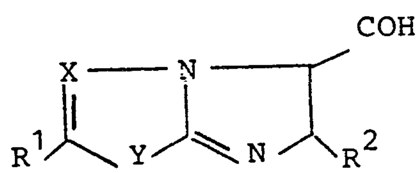

in welcher

X für N oder CH steht,

Y für S, O, NH oder N-Alkyl steht,

$R^1$ für Wasserstoff, geradkettiges, verzweigtes oder cyclisches Alkyl, Alkenyl gegebenenfalls substituiert durch Phenyl, Cyano, Hyroxyl oder Halogen steht, wobei die Kohlenstoffkette des Alkylrestes gegebenenfalls unterbrochen ist durch O, S, NH, N-Alkyl oder durch N-Aralkyl,
oder

für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Alkyl, Alkoxy, Halogen, Nitro, Trifluormethyl oder $SO_n$-Alkyl (n = 0 bis 2)
oder
für eine $SO_n$-Alkylgruppe steht, wobei n eine ganze Zahl von 0 bis 2 bedeutet,
oder

R'
|
für den Rest N steht, wobei R' und R" gleich
|
R"

oder verschieden sind und für jeweils für Wasserstoff, Aralkyl, Aryl oder Alkyl stehen, wobei die Alkylreste ihrerseits durch O, S, NH oder N-Alkyl unterbrochen sein können oder wobei R' und R" gemeinsam mit dem Stickstoffatom einen 5- bis 7-gliedrigen Ring bilden können, der seinerseits gegebenenfalls 1 oder 2 weitere Heteroatome aus der Gruppe O, S oder NH enthält, wobei der Stickstoff durch Alkyl, Aryl oder Aralkyl substituiert sein kann,
oder
für den Rest COR"' steht, wobei R"' Wasserstoff, Hydroxyl, Alkyl, Alkoxy, Alkenyl, Alkenoxy, Alkinyl, Aralkoxy, Aryloxy oder

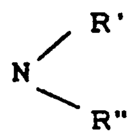

bedeutet, und wobei R' und R" die oben angegebenen Bedeutungen besitzen, und

$R^2$ für Wasserstoff, geradkettiges, verzweigtes oder cyclisches Alkyl, Alkenyl oder Alkinyl, gegebenenfalls substituiert durch Phenyl, Cyano oder Halogen steht, wobei die Kohlenstoffkette des Alkylrestes gegebenenfalls unterbrochen ist durch O, S, NH, N-Alkyl oder durch N-Aralkyl,

oder

für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Alkyl, Alkoxy, Halogen, Trifluormethyl oder $SO_n$-Alkyl (n = 0 bis 2)

oder

für eine $SO_n$-Alkylgruppe steht, wobei n eine ganze Zahl von 0 bis 2 bedeutet,

oder

für den Rest 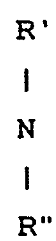 steht, wobei R' und

R" gleich oder verschieden sind und jeweils für Wasserstoff, Aralkyl oder Alkyl stehen, wobei die Alkylreste ihrerseits durch O, S oder N-Alkyl unterbrochen sein können oder wobei R' und R" gemeinsam mit dem Stickstoffatom einen 5- bis 7-gliedrigen Ring bilden können, der seinerseits gegebenenfalls 1 oder 2 weitere Heteroatome aus der durch Alkyl, Aryl oder Aralkyl substituiert sein kann.

Die erfindungsgemäßen als Ausgangsverbindungen einsetzbaren Carbonylverbindungen können analoger Weise nach folgenden bekannten Methoden hergestellt werden:

Methode a)

L. Pentimalli et al, Boll.Sci.Fac.Chim.Ind. Bologna 23, 181 (1965); s. Chem. Abstr. 63, 17848 e (1965).

Methode b)

D. Brower et al; J.Chem.Soc. 1955, 2834.

Methode c)

A. Hetzheim et al, Chem. Ber. 103, 3533 (1970).

Methode d)

H. Beyer et al, Z. Chem. 2, 152 (1962).

**Methode e)**

S. Kano, Yakugku Zasski 92, 935 (1972).

Die erfindungsgemäßen Carbonylverbindungen sind wertvolle Zwischenprodukte für die Herstellung von Arzneimitteln, insbesondere von Antihypertensiva, Diuretika und Uricosurica.

Ihre Überführung in solche Wirkstoffe geschieht wie nachfolgend erläutert:

Erfindungsgemäße Carbonylverbindungen der allgemeinen Formel

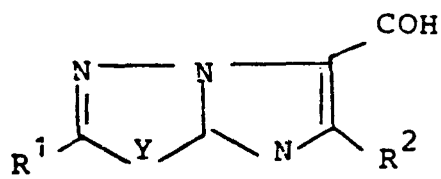

in welcher

X, Y, $R^1$ und $R^2$ die oben angegebene Bedeutung haben,

werden mit Malonsäuren der allgemeinen Formel

$$HOOC-\underset{\displaystyle R^3}{\underset{|}{CH}}-COOH$$

in welcher

$R^3$ für Wasserstoff, Alkyl mit 1 bis 6 Kohlenstoffatomen, gegebenenfalls unterbrochen durch Sauerstoff und gegebenenfalls substituiert durch Fluor, Chlor oder Cyano steht

oder
für Cyano, Nitro oder für den Rest COR"' steht, wobei R"' Alkyl oder Alkoxy mit jeweils bis zu 4 Kohlenstoffatomen bedeutet,
oder
für $SO_n$-Alkyl (n = 0 oder 2) mit 1 bis 4 Kohlenstoffatomen im Alkylteil steht,

in Gegenwart von inerten organischen Lösungsmitteln und gegebenenfalls in Gegenwart von Kondensationsmitteln kondensiert. Man erhält dabei Alkensäuren der allgemeinen Formel

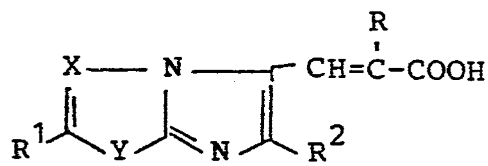

die bei der Umsetzung mit Aminen, gegebenenfalls nach Aktivierung der Carboxylgruppe über das entsprechende Säurehalogenid, in üblicher Weise, gegebenenfalls in Gegenwart von inerten organischen Lösungsmitteln bei Temperaturen zwischen 20 und 150°C, zu den entsprechenden Amiden führen. Diese besitzen die oben genannten wertvollen pharmakologischen Eigenschaften.

Von den erfindungsgemäßen Zwischenprodukten sei insbesondere der 2-Methyl-6-phenyl-imidazo[2,1-b]-1,3,4-thiadiazol-5-carbaldehyd der Formel

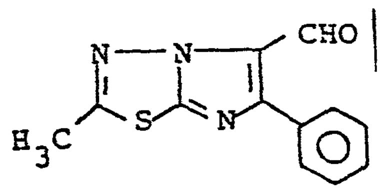

genannt. Überführt man ihn wie oben beschrieben in das Amid der Formel

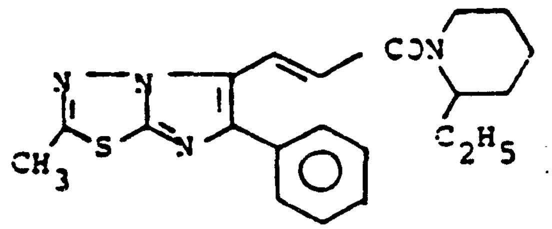

so erhält man ein hochwirksames Diuretikum. Setzt man dagegen die entsprechende, aus US-PS 3 615 639, Beispiel 33, bekannte p-Nitroverbindung der Formel

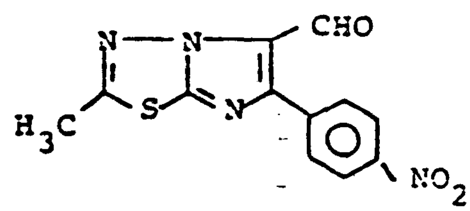

in analoger Weise um, so erhält man die unwirksame Verbindung der Formel

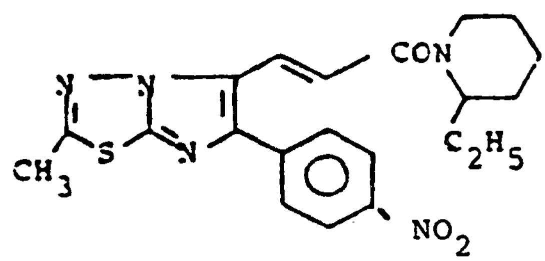

Die erfindungsgemäßen Verbindungen sind somit bekannten Verbindungen ähnlicher Struktur weit überlegen.

**Beispiel 1**

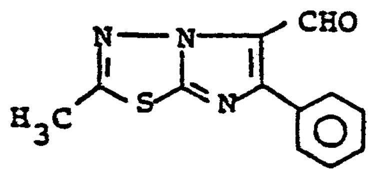

**2-Methyl-6-phenyl-imidazo[2,1-b]-1,3,4-thiadiazol-5-carbaldehyd**

Unter Kühlung werden zu 180 ml Dimethylformamid 60 ml Phosphoroxichlorid zugetropft. Dann gibt man weitere 400 ml Dimethylformamid und 130 g (0,6 Mol) 2-Methyl-6-phenyl-imidazo[2,1-b]1,3,4-thiadiazol zu und erwärmt 2 Stunden auf 100°C. Der Ansatz wird auf 3 kg Eis gegeben, mit 410 g 40 %iger Natronlauge versetzt und schnell auf 90°C erhitzt. Nach 5 Minuten wird abgekühlt und abgesaugt. Ausbeute 132 g (90 % der Theorie) vom Fp. 152 bis 153°C.

In analoger Weise wurden die Beispiele der nachfolgenden Tabelle 1 durch Umsetzung jeweils äquivalenter Mengen der Reaktionspartner hergestellt.

Tabelle 1

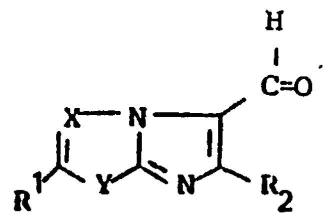

| Beispiel-Nr. | X | Y | $R^1$ | $R^2$ | Fp. °C | Ausbeute % d.Th. |
|---|---|---|---|---|---|---|
| 2 | CH | S | H | $C_6H_5$ | 128-9 | 25 |
| 3 | N | S | $CF_3$ | $C_6H_5$ | 186-8 | 32 |
| 4 | N | S | $C_2H_5$ | $C_6H_5$ | 101-2 | 28 |
| 5 | N | S | $CH_2$- 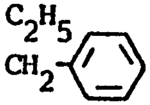 | $C_6H_5$ | 140-2 | 40 |
| 6 | N | S | $CH=CH-CH_3$ | $C_6H_5$ | 164-5 | 33 |
| 7 | N | S | 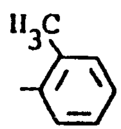 | $C_6H_5$ | 149 | 29 |
| 8 | N | S | H | $C_6H_5$ | 234-38 | 34 |
| 9 | N | O | $CH_3$ | $C_6H_5$ | 165 | 38 |
| 10 | N | NH | $CH_3$ | $C_6H_5$ | 270 | 36 |

Tabelle 1 (Fortsetzung)

| Bei-spiel-Nr. | X | Y | $R^1$ | $R^2$ | Fp. °C | Ausbeute % d.Th. |
|---|---|---|---|---|---|---|
| 11 | N | S | $CH_3$ | 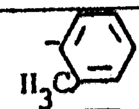 | 109-11 | 56 |
| 12 | N | S | $CH_3$ | 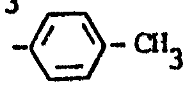 | 139-41 | 64 |
| 13 | N | S | $CH_3$ | 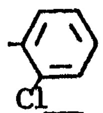 | 120-22 | 48 |
| 14 | N | S | $CH_3$ | 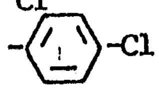 | 175-77 | 71 |
| 15 | N | S | $CH_3$ | 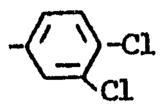 | 203-05 | 69 |
| 16 | N | S | $CH_3$ | 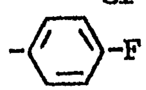 | 168-70 | 66 |
| 17 | N | S | $CH_3$ | 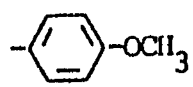 | 160-62 | 65 |

Tabelle 1 (Fortsetzung)

| Bei-spiel-Nr. | X | Y | $R^1$ | $R^2$ | Fp. °C | Ausbeute % d.Th. |
|---|---|---|---|---|---|---|
| 18 | N | S | $SO_2CH_3$ | $C_6H_5$ | 250 | 52 |
| 19 | N | S | $(CH_2)_2-O-C_2H_5$ | $C_6H_5$ | 57 | 40 |
| 20 | N | S | $(CH_2)_2-S-CH_3$ | $C_6H_5$ | 83 | 66 |
| 21 | N | S | $CO_2n-C_4H_9$ | $C_6H_5$ | 74 | 85 |
| 22 | N | S | 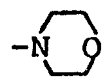 | $C_6H_5$ | 180-82 | 83 |
| 23 | N | S | 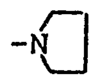 | $C_6H_5$ | 200-02 | 95 |
| 24 | N | S | 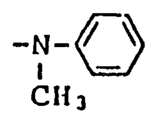 | $C_6H_5$ | 142-44 | 87 |
| 25 | N | S | 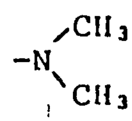 | $C_6H_5$ | 167-69 | 96 |

Tabelle 1 (Fortsetzung)

| Beispiel-Nr. | X | Y | $R^1$ | $R^2$ | Fp. °C | Ausbeute % d.Th. |
|---|---|---|---|---|---|---|
| 26 | N | S | 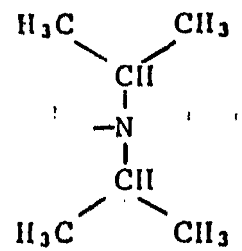 | $C_6H_5$ | 128-30 | 91 |
| 27 | N | S | $-CH_3$ | 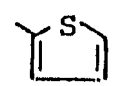 | 184-86 | 73 |
| 28 | N | S | $-CH_3$ | 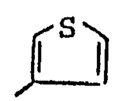 | 181-83 | 95 |

Patentansprüche

1. Imidazoazolcarbonylverbindungen der allgemeinen Formel

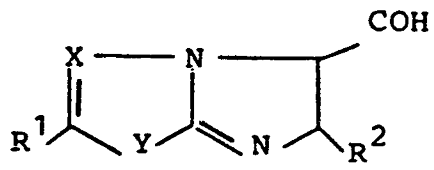

in welcher

X für N oder CH steht,

Y für S, O, NH oder N-Alkyl steht,

$R^1$ für Wasserstoff, geradkettiges, verzweigtes oder cyclisches Alkyl, Alkenyl oder Alkinyl, gegebenenfalls substituiert durch Phenyl, Cyano, Hydroxyl oder Halogen steht, wobei die Kohlenstoffkette des Alkylrestes gegebenenfalls unterbrochen ist durch O, S, NH, N-Alkyl oder durch N-Aralkyl,
oder
für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Alkyl, Alkoxy, Halogen, Nitro, Trifluormethyl oder $SO_n$-Alkyl (n = 0 bis 2)
oder
für eine $SO_n$-Alkylgruppe steht, wobei n eine ganze Zahl von 0 bis 2 bedeutet,

für den Rest 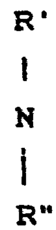 steht, wobei R' und R" gleich oder verschieden sind und jeweils für Wasserstoff, Aralkyl, Aryl oder Alkyl stehen, wobei die Alkylreste ihrerseits durch O, S, NH oder N-Alkyl unterbrochen sein können oder wobei R' und R" gemeinsam mit dem Stickstoffatom einen 5- bis 7-gliedrigen Ring bilden können, der seinerseits gegebenenfalls 1 oder 2 weitere Heteroatome aus der Gruppe O, S oder NH enthält, wobei der Stickstoff durch Alkyl, Aralkyl substituiert sein kann,

oder

für den Rest COR"' steht, wobei R"' Wasserstoff, Hydroxyl, Alkyl, Alkoxy, Alkenyl, Alkenoxy, Alkinyl, Aralkoxy, Aryloxy oder

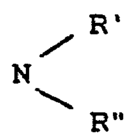

bedeutet, und wobei R' und R" die oben angegebenen Bedeutungen besitzen, und

$R^2$ für Wasserstoff, geradkettiges, verzweigtes oder cyclisches Alkyl, Alkenyl oder Alkinyl, gegebenenfalls substituiert durch Phenyl, Cyano oder Halogen steht, wobei die Kohlenstoffkette des Alkylrestes gegebenenfalls unterbrochen ist durch O, S, NH, N-Alkyl oder durch N-Aralkyl,

oder

für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Alkyl, Alkoxy, Halogen, Trifluormethyl oder $SO_n$-Alkyl (n = 0 bis 2)

oder

für eine $SO_n$-Alkylgruppe steht, wobei n eine ganze Zahl von 0 bis 2 bedeutet,

oder

für den Rest 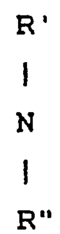 stehen, wobei R' und R" gleich oder verschieden sind und jeweils für Wasserstoff, Aralkyl oder Alkyl stehen, wobei die Alkylreste ihrerseits durch O, S oder N-Alkyl unterbrochen sein können oder wobei R' und R" gemeinsam mit dem Stickstoffatom einen 5- bis 7-gliedrigen Ring bilden können, der seinerseits gegebenenfalls 1 oder 2 weitere Heteroatome aus der Gruppe O, S oder N enthält, wobei der Stickstoff durch Alkyl, Aryl oder Aralkyl substituiert sein kann,

oder

für den Rest COR"' steht, wobei R"' Wassserstoff, Hydroxyl, Alkyl, Alkoxy, Alkenyl, Alkenoxy, Alkinyl, Aralkoxy, Aryloxy oder

0158012

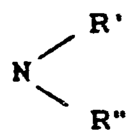

bedeutet, und wobei R' und R" die oben angegebenen Bedeutungen besitzen.

2. Imidazoazolcarbonylverbindungen der allgemeinen Formel

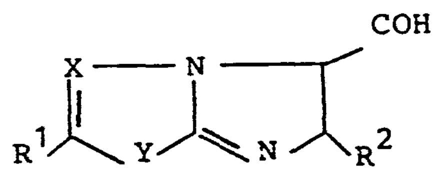

in welcher

X für N oder CH steht,

Y für S, O, NH oder N-Alkyl mit 1 bis 4 Kohlenstoffatomen steht,

$R^1$ für Wasserstoff, geradkettiges oder verzweigtes oder cyclisches Alkyl, Alkenyl oder Alkinyl mit bis zu 6 Kohlenstoffatomoen, welche gegebenenfalls substituiert ist durch Phenyl, Cyano, Hydroxyl, Methyl, Ethyl, Methoxy, Ethoxy, Trifluormethyl, Fluor, Chlor oder Brom steht, wobei die Kohlenstoffkette

0158012

ist durch O, S, NH, N-Alkyl mit 1 bis 4 Kohlenstoffatomen oder durch N-Benzyl,
oder
für einen Phenyl-, Naphthyl, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Nitro, Trifluormethyl, Fluor, Chlor, Brom, Alkyl, Alkoxy oder $SO_n$-Alkyl (n 0, 1 oder 2), wobei die genannten Alkyl- und Alkoxyreste jeweils 1 bis 4 Kohlenstoffatome enthalten,
oder
für eine $SO_n$-Alkylgruppe steht, wobei n und Alkyl die oben angegebene Bedeutung haben,
oder

```
                R'
                |
für den Rest    N   steht, wobei R' und R" gleich
                |
                R"
```

oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1-4 C-Atome) unterbrochen sein können oder wobei R' und R" gemeinsam mit dem Stickstoffatom einen 5- bis 7-gliedrigen Ring bilden, der

seinerseits gegebenenfalls 1 oder 2 weitere gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,
oder
für den Rest COR"' steht, wobei R"' Wasserstoff, Hydroxyl, Alkyl, Alkoxy mit jeweils 1 bis 6 C-Atomen, Phenoxy, Benzyloxy, Alkenoxy mit bis zu 4 C-Atomen, oder den Rest R' bedeutet, wobei R' und R" die oben

|
N
|
R"

angegebene Bedeutung haben, und

$R^2$ für Wasserstoff, geradkettiges oder verzweigtes oder cyclisches Alkyl, Alkenyl oder Alkinyl mit bis zu 6 Kohlenstoffatomen, welches gegebenenfalls substituiert ist durch Phenyl, Cyano, Hydroxyl, Methyl, Ethyl, Methoxy, Ethoxy, Trifluormethyl, Fluor, Chlor oder Brom steht, wobei die Kohlenstoffkette des Alkylrestes gegebenenfalls unterbrochen ist durch O, S, NH, N-Alkyl mit 1 bis 4 Kohlenstoffatomen oder durch N-Benzyl,
oder

für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Trifluormethyl, Fluor, Chlor, Brom, Alkyl, Alkoxy oder $SO_n$-Alkyl (n = 0, 1 oder 2), wobei die genannten Alkyl- und Alkoxyreste jeweils 1 bis 4 Kohlenstoffatomen enthalten,

oder

für eine $SO_n$-Alkylgruppe steht, wobei n und Alkyl die oben angegebene Bedeutung haben,

oder

```
              R'
              |
für den Rest  N   steht, wobei R' und R" gleich
              |
              R"
```

oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1-4 C-Atome) unterbrochen sein können oder wobei R' und R" gemeinsam mit dem Stickstoffatom einen 5- bis 7-gliedrigen Ring bilden, der seinerseits gegebenenfalls 1 oder 2 weitere gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,
oder
für den Rest COR"' steht, wobei R"' Wasserstoff, Hydroxyl, Alkyl, Alkox mit jeweils 1 bis 6 C-Atomen, Phenoxy, Benzyloxy, Alkenoxy mit bis zu 4 C-Atomen, oder den Rest

R'
|
-N
|
R"

angegebene Bedeutung haben.

3. 2-Methyl-6-phenyl-imidazo[2,1-b]-1,3,4-thiadiazol-5-carbaldehyd.

4. Verwendung der Imidazoazolcarbonylverbindungen gemäß Anspruch 1 als Zwischenprodukte für die Herstellung von Arzneimitteln.

5. Verfahren zur Herstellung von Imidazoazolcarbonylverbindungen der allgemeinen Formel

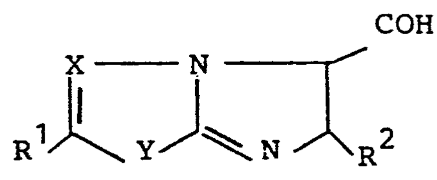

in welcher

X für N oder CH steht,

Y für S, O, NH oder N-Alkyl mit 1 bis 4 Kohlenstoffatomen steht,

$R^1$ für Wasserstoff, geradkettiges oder verzweigtes oder cyclisches Alkyl, Alkenyl oder Alkinyl mit bis zu 6 Kohlenstoffatomen, welches gegebenenfalls substituiert ist durch PHenyl, Cyano, Hydroxyl, Methyl, Ethyl, Methoxy, Ethoxy, Trifluormethyl, Fluor, Chlor oder Brom steht, wobei die Kohlenstoffkette des Alkylrestes gegebenenfalls unterbrochen ist durch O, S, NH, N-Alkyl mit 1 bis 4 Kohlenstoffatomen oder durch N-Benzyl,
oder
für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleiche oder verschiedene Substituenten aus der Gruppe Nitro, Trifluormethyl, Fluor, Chlor, Brom, Alkyl, Alkoxy oder $SO_n$-Alkyl (n = 0, 1 oder 2), wobei die genannten Alkyl- und Alkoxyreste jeweils 1 bis 4 Kohlenstoffatome enthalten,
oder

für einen $SO_n$-Alkylgruppe steht, wobei n und Alkyl die oben angegebene Bedeutung haben, oder

R'
|
für den Rest N steht, wobei R' und R" gleich
|
R"

oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1-4 C-Atome) unterbrochen sein können oder wobei R' und R" gmeinsam mit dem Stickstoffatom einen 5- bis 7-gliedrigen Ring bilden, der seinerseits gegebenenfalls 1 oder 2 weitere gleiche oder verschiedene Heteroatome aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,
oder
für den Rest COR"' steht, wobei R"' Wasserstoff, Hydroxyl, Alkyl, Alkoxy mit jeweils 1 bis 6 C-Atomen, Phenoxy, Benzyloxy, Alkenoxy

mit bis zu 4 C-Atomen, oder den Rest
R' bedeutet, wobei R' und R" die oben
|
N
|
R"

angegebene Bedeutung haben, und

$R^2$ für Wasserstoff, geradkettiges oder verzweigtes oder cyclisches Alkyl, Alkenyl oder Alkinyl mit bis zu 6 Kohlenstoffatomen, welches gegebenenfalls substituiert ist durch Phenyl, Cyano, Hydroxyl, Methyl, Ethyl, Methoxy, Ethoxy, Trifluormethyl, Fluor, Chlor oder Brom steht, wobei die Kohlenstoffkette des Alkylrestes gegebenenfalls unterbrochen ist durch O, S, NH, N-Alkyl mit 1 bis 4 Kohlenstoffatomen oder durch N-Benzyl,
oder
für einen Phenyl-, Naphthyl-, Furyl-, Thienyl-, Pyrimidyl-, Pyrazinyl-, Chinolyl-, Isochinolyl- oder Pyridylring steht, der gegebenenfalls substituiert ist durch 1, 2 oder 3 gleich oder verschiedene Substituenten aus der Gruppe Trifluormethyl, Fluor, Chlor, Brom, Alkyl, Alkox oder $SO_n$-Alkyl- und Alkoxyreste jeweils 1 bis 4 Kohlenstoffatome enthalten,
oder

0158012

für eine $SO_n$-Alkylgruppe steht, wobei n und Alkyl die oben angegebene Bedeutung haben,
oder

R'
|
für den Rest -N steht, wobei R' und R" gleich
|
R"

oder verschieden sind und jeweils für Wasserstoff, Benzyl, Phenyl oder geradkettiges, verzweigtes oder cyclisches Alkyl mit 1 bis 6 Kohlenstoffatomen stehen, wobei die Alkylreste ihrerseits durch O, S oder N-Alkyl (1-4 C-Atome) unterbrochen sein können oder wobei R' und R" gemeinsam mit dem Stickstoffatom einen 5- bis 7-gliedrigen Ring bilden, der seinerseits gegebenenfalls aus der Gruppe Sauerstoff, Schwefel oder Stickstoff enthalten kann, wobei der Stickstoff gegebenenfalls durch Wasserstoff, Alkyl mit 1 bis 4 Kohlenstoffatomen, Phenyl oder Benzyl substituiert ist,
oder
für den Rest COR"' steht, wobei R"' Wasserstoff, Hydroxyl, Alkyl, Alkoxy mit jeweils 1 bis 6 C-Atomen, Phenoxy, Benzyloxy, Alkenoxy mit bis zu 4 C-Atomen oder den Rest

R'
|
-N
|
R"

angegebenene Bedeuutng haben,

dadurch gekennzeichnet, daß man Verbindungen der Formel

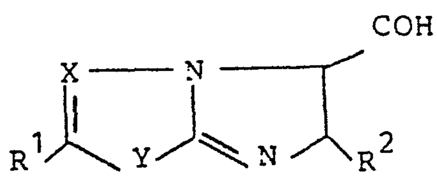

mit Dimethylformamid und Phosphoroxichlorid erwärmt.

0158012

Europäisches Patentamt

# EUROPÄISCHER RECHERCHENBERICHT

Nummer der Anmeldung

EP 85 10 0843

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | IL FARMACO - ED. SC., Band 35, Fasc. 7, 1980, Seiten 573-80, Pavia, IT; A. ANDREANI u.a.: "Composti a presunta attivita antitumorale" * Seite 574, Verbindungen II,III * | 1 | C 07 D 513/04<br>C 07 D 498/04<br>C 07 D 487/04 //<br>(C 07 D 513/04<br>C 07 D 285:00<br>C 07 D 235:00 )<br>(C 07 D 513/04<br>C 07 D 277:00<br>C 07 D 235:00 )<br>(C 07 D 498/04<br>C 07 D 271:00<br>C 07 D 235:00 )<br>(C 07 D 487/04<br>C 07 D 249:00<br>C 07 D 235:00 )<br>(C 07 D 498/04<br>C 07 D 263:00<br>C 07 D 235:00 )<br>-/- |
| X | CHEMICAL ABSTRACTS, Band 87, 1977, Seite 596, Nr. 152204d, Columbus, Ohio, US; & SU - A - 562 554 (ROSTOV STATE UNIVERSITY) 25.06.1977 | 1 | |
| X | JOURNAL OF ORGANIC CHEMISTRY, Band 38, Nr. 10, 1973, Seiten 1955-57, Washington, US; L.F. MILLER u.a.: "1H-Imidazo[1,2-a]imidazoles. II. The chemistry of 1,6-dimethyl-1H-imidazo[1,2-a]imidazole" * Seite 1956, Verbindung 4; Seite 1957, Spalte 1, Zeilen 20-32 * | 1 | RECHERCHIERTE SACHGEBIETE (Int. Cl.4)<br>C 07 D 513/00<br>C 07 D 498/00<br>C 07 D 487/00 |
| X | US-A-3 615 639 (EASTMAN KODAK) * Beispiele 28,33 * | 1 | |
| | --- -/- | | |

Der vorliegende Recherchenbericht wurde für alle Patentansprüche erstellt.

| Recherchenort | Abschlußdatum der Recherche | Prüfer |
|---|---|---|
| DEN HAAG | 19-06-1985 | ALFARO I. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82

Europäisches Patentamt

# EUROPÄISCHER RECHERCHENBERICHT

0158012

Nummer der Anmeldung

EP 85 10 0843

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 93, 1980, Seite 638, Nr. 149290x, Columbus, Ohio, US; V.A. ANISIMOVA u.a.: "Chloral as a formylation agent for some bridging hetero systems" & KHIM. GETEROTSIKL. SOEDIN. 1980, (4), 528-37 * Zusammenfassung und SUBSTANCE INDEX, Seite 26315, Spalte 2, Zeilen 65-67 * ----- | 1 | (C 07 D 487/04 C 07 D 235:00 C 07 D 235:00 ) |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| DEN HAAG | 19-06-1985 | ALFARO I. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82